# EUROPEAN PATENT APPLICATION

(11) **EP 1 346 989 A1**
(43) Date of publication of application: **24.09.2003**
(21) Application number: 02252047.2
(22) Date of filing: 21.03.2002
(51) Int. Cl.: C07D 307/87

(54) **Improved process for the preparation of citalopram and its hydrobromide**

(71) Applicant: Jubilant Organosys Limited, Noida 201 301, Uttar Pradesh (IN)
(72) Inventor: Babu, Ambati Narahari, Mysore-570 023, Karnataka (IN); Goud, Vuddamari Srinivas, Mysore-570 023, Karnataka (IN); Gaonkar, Santhosh Laxman, Mysore-570 301, Karnataka (IN); Thomas, Saji D., Mysore-570 023, Karnataka (IN); Manjunatha, Sulur G., Mysore-570 023, Karnataka (IN); Kulkami, Ashok Krishna, Mysore-570 023 Karnataka (IN)
(74) Representative: Coates, Ian Harold

(57) **Abstract**

A process is described for the preparation of citalopram (an anti-depressant drug), which process comprises the C-alkylation of 1-(4'-fluorophenyl)-1,3-dihydro*iso*benzofuran-5-carbonitrile (5-cyanophthalane) with 3-dimethylaminopropylchloride in the presence of potassium tertiary butoxide. Suitably, the alkylation is carried out in the presence of dimethylsulphoxide (DMSO).

The citalopram thereby produced can be isolated as a crystalline solid in one step from the reaction mixture by adding an equal volume of a water-miscible solvent, such as IPA, to the mixture. A process is also described for preparing citalopram hydrobromide, which process comprises reacting citalopram (base) with aqueous hydrobromic acid, such as at pH 1-3.

## Description

The present invention relates to an improved process for the preparation of citalopram (1-[3-(dimethylamino)propyl]-1-(4-fluorophenyl)-1,3-dihydro-5-*iso*benzofurancarbonitrile) by the C-alkylation of cyanophthalane (1-(4'-fluorophenyl)-1,3-dihydroisobenzofuran-5-carbonitrile) with 3-dimethylaminopropylchloride in the presence of a base. The invention further relates to isolation of highly pure crystalline citalopram base in a single step from the reaction mixture and an improved procedure for converting citalopram base to its hydrobromide salt.

Citalopram and its pharmaceutically acceptable acid addition salts, such as its hydrogen bromide salt (Formula (I)) as described in US patent specification no. 4 136 193, are anti-depressant drugs with few side effects.

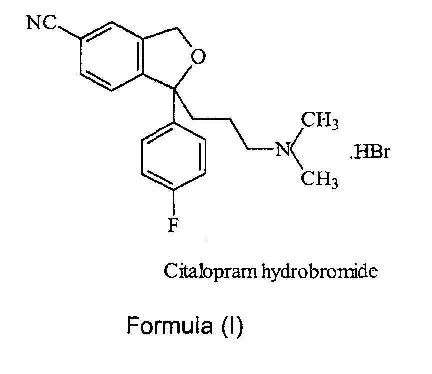

Various processes for the preparation of citalopram have been described in the prior art. For example, US patent specification no. 4 136 193 describes the C-alkylation of cyanophthalane with 3-dimethylaminopropylchloride, using sodium hydride as a base in a dimethylsulphoxide (DMSO) medium (Scheme - 1). The reaction mixture is poured into ice water and extracted with ether. Then, after standard acid-base work-up, citalopram base is isolated as an impure oil. The isolated oil is purified by high vacuum distillation, (0.03mm at 175-180°C) and then converted to the hydrobromide salt.

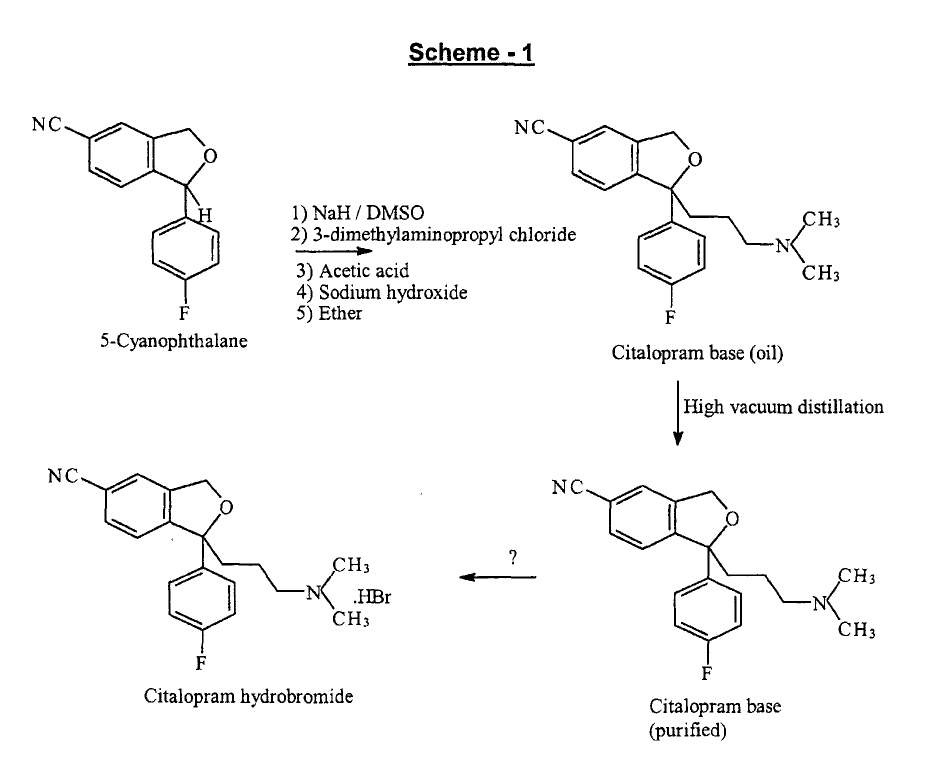

A drawback of this process is the necessity to handle sodium hydride, in particular, in the presence of a dimethylsulphoxide medium. Sodium hydride is known to form an explosive mixture with dimethylsulphoxide, and ignites spontaneously in air; is flammable when exposed to heat or flame; and reacts violently with water [Vogel's Textbook of Practical Organic Chemistry, 5^{th} edition, 412; Sax's Dangerous Properties of Industrial Materials; 8th edition, 3085].

Another drawback of this process is in the need to purify the oily citalopram base using high vacuum distillation (0.03mm) at 175-181°C. Achieving such a very high vacuum at plant level is difficult and hence the process described is not easily transferable to the commercial scale. Apart from these constraints, citalopram base having a cyano group at the 5^{th} position of the bicyclic ring system may decompose during high vacuum distillation at high temperature to form citalopram carboxamide as an impurity, resulting in poor quality and yield.

Another process, as described in PCT patent specification no. WO 98/19511, is the C-alkylation of cyanophthalane with 3-dimethylaminopropylchloride in the presence of a strong base (such as n-butyl lithium) and diisopropylamine in a dimethoxyethane medium at -50°C (Scheme - 2). After completion of the reaction, the reaction mixture is poured into ice water and extracted with toluene. After standard acid-base work-up using toluene as solvent, citalopram base is isolated as an oil and then converted to its hydrobromide salt.

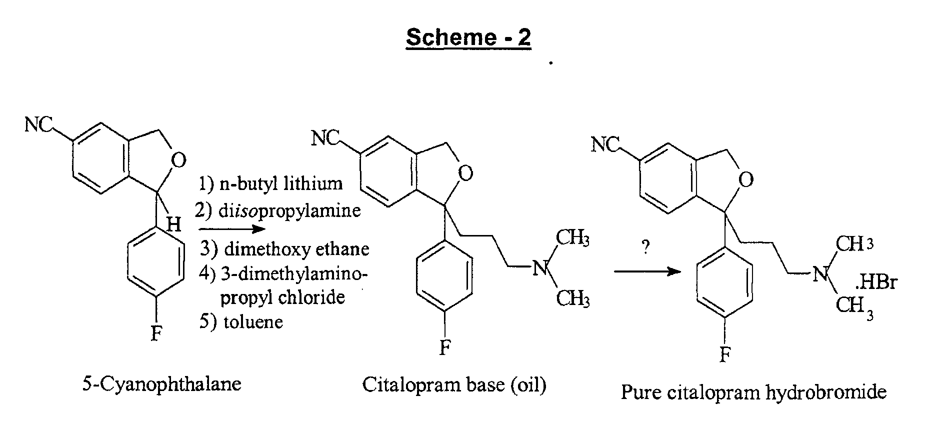

The main drawback of this process is in the necessity to handle n-butyl lithium, which is highly pyrophoric; a solution of n-butyl lithium greater than 20% will ignite spontaneously in air. The second drawback of the process is the reaction temperature, since -50°C to difficult to achieve at plant level.

Furthermore, although the above two patent specifications mention that citalopram hydrobromide can be made from the base by standard procedures, a specific procedure is not described.

German patent specification no. DE 20 007 303 discloses the isolation of citalopram base, as a solid, from citalopram hydrobromide and the isolated base is then converted into the desired salt. Pure citalopram hydrobromide is dissolved in 5 volumes of water and the pH is adjusted to about 10 with 6N sodium hydroxide. The citalopram base is then extracted into a non-polar organic solvent, such as toluene. The toluene is distilled off under reduced pressure and the resulting residue is triturated with n-heptane to precipitate citalopram base as a solid. The solid is then filtered to produce crystalline citalopram base.

The main disadvantage of this process is in the preparation of citalopram hydrobromide from the base and then converting back to solid citalopram base. Then the solid base again needs to be converted to its corresponding hydrobromide salt. Apart from the to and fro conversions involving too many steps, as mentioned above, prolonged heating of citalopram base may increase the carboxamide impurity, resulting in poor quality citalopram.

Citalopram hydrobromide has also been made by a different approach, as described in US patent specification no. 4 650 884. According to the method described here, the citalopram base, isolated as an oil, is diluted with acetone, and hydrogen bromide gas is introduced over a period of two hours to adjust the pH to 3.0 and thereby to produce crude citalopram hydrobromide. The crude citalopram hydrobromide is then purified by repeated crystallizations, in water (1^{st} purification), then methanol - isopropanol (2^{nd} purification), followed by methanol-acetone and hexane (3^{rd} purification), to result in the desired quality of citalopram hydrobromide. This process therefore involves repeated crystallizations, which makes it an unattractive one at plant level. Furthermore, the generation and handling of anhydrous hydrogen bromide is preferably to be avoided.

The present invention therefore provides: (a) a safer and simpler process for the preparation of pure citalopram hydrobromide *via* the C-alkylation of cyanophthalane with 3-dimethylaminopropylchloride using potassium tertiary butoxide; (b) a process for the isolation of crystalline citalopram base from the reaction mixture using water and a water-miscible solvent; and (c) an advantageous procedure for converting citalopram base to citalopram hydrobromide using aqueous HBr.

According to a first aspect the present invention, therefore, the C-alkylation of 1-(4'-fluorophenyl)-1,3-dihydro*iso*benzofuran-5-carbonitrile (5-cyanophthalane) with 3-dimethylaminopropyl-chloride is carried out in the presence of a base, characterized in that the base is potassium tertiary butoxide (Scheme - 3).

The reaction is preferably carried out in the presence of a suitable solvent, such as an oxygenated solvent, such as dimethylsulphoxide (DMSO) or dimethoxyethane, but most preferred is DMSO.

After the reaction is completed, the reaction mixture can be poured on to ice water and extracted with a suitable solvent, such as non-polar, organic solvent, *eg* toluene, methylenedichloride, chloroform, ethylacetate and diethyl ether, but most preferred is toluene. It has surprisingly been found unnecessary to carry out vacuum distillation to purify the oily citalopram base prepared using potassium t-butoxide. Using a standard acid/base work-up, sufficiently pure citalopram base is isolated as an oil, which can then be converted to a salt, such as the hydrogen bromide salt, by known methods.

For comparison, as described in the patent US patent specification no. 4 136 193, sodium hydride was used as the base, instead of potassium tert-butoxide. The quality of the citalopram hydrobromide made by using potassium tert-butoxide was found (Example 1 and Scheme - 3) to be superior in quality and yield to the one made by using sodium hydride. Citalopram hydrobromide prepared by using sodium hydride needed one or two purification steps to match the quality of citalopram hydrobromide prepared by using potassium tert-butoxide.

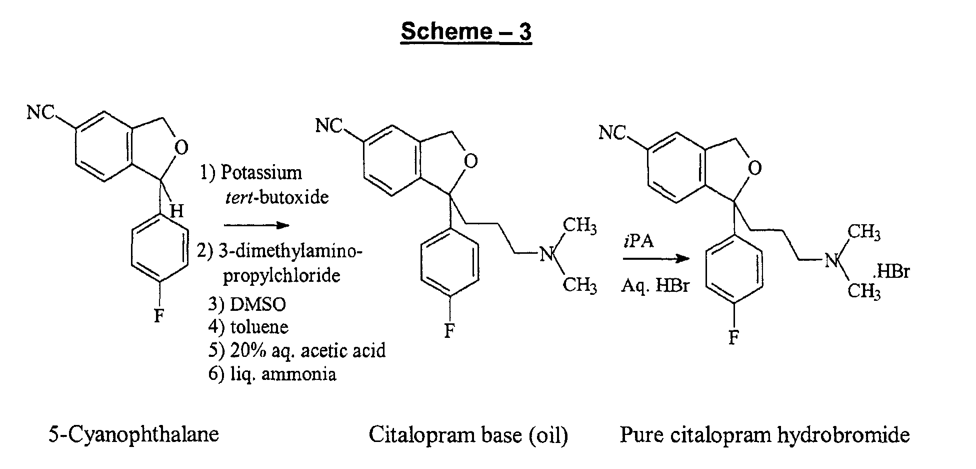

A typical acid work-up comprises extraction of the organic (preferably, toluene) layer with an aqueous acid, such as a mineral acid (*eg* hydrochloric or hydrobromic acids) or, preferably, a weaker organic acid, such as formic, oxalic or acetic acid. Most preferred is to use 20% aqueous acetic acid.

More preferred is to isolate citalopram base as a crystalline solid of high purity directly from the C-alkylation reaction mixture (Examples 2 & 3 and Scheme - 4). In this second aspect of the invention, to an aqueous mixture of oily citalopram is added an equal volume of a water-miscible solvent. Suitable water-miscible solvents comprise: alcohols, such as methanol, ethanol and *iso*propanol; ketones, such as acetone; dimethylformamide (DMF) and/or dimethylsulphoxide (DMSO), especially *iso*propylalcohol (IPA)). The oily citalopram is preferably that resulting from the C-alkylation of 1-(4'-fluorophenyl)-1,3-dihydro*iso*benzofuran-5-carbonitrile (5-cyanophthalane) with 3-dimethylaminopropyl-chloride in the presence of a base followed by (preferably acetic) acid work-up, more preferably using the process of the first aspect of this invention, as described above.

Then, a typical base work-up comprises: the pH of the mixture is carefully adjusted to about 8.5 - 9 since, at higher pH, impurities start precipitating and, at lower pH, the yield is lower. Suitable bases for adjusting the pH include aqueous ammonia, sodium hydroxide, potassium hydroxide and sodium or potassium carbonates, but preferably a mild base such as aqueous ammonia is used. The reaction is preferably carried out at below ambient temperature (*ie* below about 25 °C), more preferably between 10-15°C; at higher temperatures, yield is reduced because of the solubility and, at lower temperatures, impurity also precipitates. The mixture is then stirred, such as for additional 3-5 hours, to precipitate citalopram completely. The crystalline citalopram base can be filtered with aqueous water-miscible solvent, preferable IPA, and optionally may be washed with a washing agent to remove the water-miscible solvent, such as a hydrocarbon, *eg* a C₅₋₇ alkane or cycloalkane, preferably hexane.

By this method, high purity crystalline citalopram base can be isolated in a single step directly from the alkylation reaction mixture.

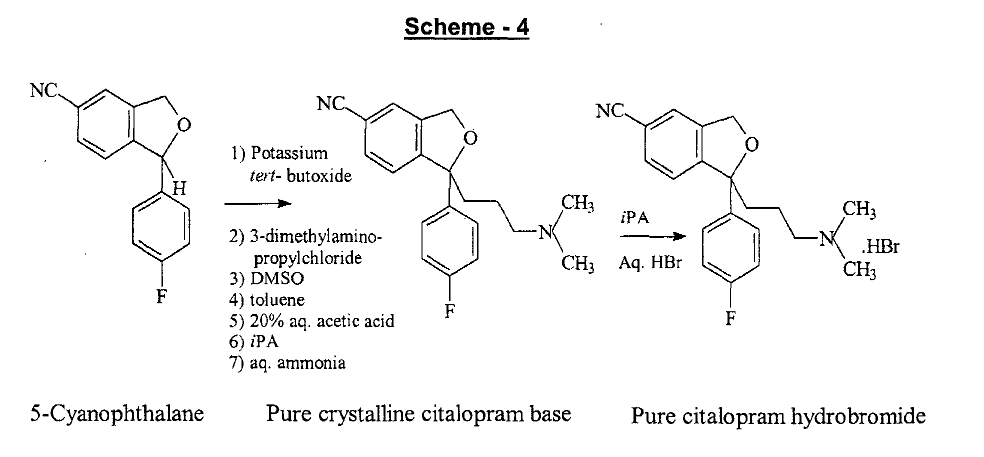

In a third aspect, the present invention provides a very simple procedure for making citalopram hydrobromide from citalopram base (Examples 1(c) and 4). The base (preferably in the form of a crystalline solid, especially when produced according to the first and/or second aspects of this invention) is dissolved in a water-miscible solvent (such as those mentioned above, preferably isopropanol) and the pH is then adjusted to acidic, preferably to pH 1-3, especially pH 1-2, with aqueous hydrobromic acid at reduced temperature (*ie* below ambient), preferably 5-10°C, for reasons given above. The mixture is then preferably stirred for an additional 5-6 hours, and the solid formed is filtered and washed with cold (0-5 °C) solvent and optionally with a wash agent such as hexane, as described above, to afford high purity crystalline citalopram hydrobromide.

The following Examples further illustrate the present invention. In each, the citalopram base was characterised by IR, ¹H-NMR, HPLC and melting point, Purity was determined by HPLC and found to be in excess of 99%.

1-(4'-Fluorophenyl)-1,3-dihydroisobenzofuran-5-carbonitrile (cyanophthalane) is prepared either by using commercially available 5-bromophthalide as per the process described in US patent specification no. 4 136 193 or by the use of commercially available 5-cyanophthalide as per the process described in PCT patent specification no. WO 98/19511.

### Example 1: Preparation of Citalopram Hydrobromide using Potassium tertiary Butoxide as a Base.

(a) 72 grams of potassium tertiary butoxide are dissolved in 575ml of dimethylsulphoxide at 60-70°C under a nitrogen atmosphere. To the resulting solution potassium salt of dimethylsuphoxide, 96 g of 1-(4'-fluorophenyl)-1,3-dihydro*iso*benzofuran-5-carbonitrile, dissolved in 150ml of dimethylsulphoxide, is added dropwise over a period of 10 min at 25-30°C. The mixture is then stirred for additional 10 min at 25-30°C. 53 g of 3-dimethylaminopropylchloride in 25 ml of dimethylsulphoxide are added quickly and the reaction mixture is then heated to 40°C and maintained for 50 min. The reaction mixture is then poured into ice-water and extracted with toluene. The toluene phase is extracted with 200ml of 20% aqueous acetic acid (40ml acetic acid and 160ml water).
(b) The acetic acid solution is cooled to 5-10°C and the pH is adjusted to 8.5-9.0 using aqueous ammonia (85ml), whilst maintaining the temperature between 5-10°C. The aqueous phase is then extracted with toluene (3 x 300ml) and the toluene layer is washed with water (200ml). The toluene layer is treated with activated carbon and dried over anhydrous sodium sulphate. The toluene is then distilled off under reduced pressure at below 60°C to produce citalopram base as an oil.
(c) The oil is then dissolved in 500ml of *iso*propyl alcohol. The pH is then adjusted between 2.0 to 3.0 using aqueous hydrobromic acid (47%) at 5-10°C. The precipitated citalopram hydrobromide solid is then filtered and washed with 100ml chilled (0-5°C) *iso*propyl alcohol.

Yield: 110-115g
Purity by HPLC 99.5%
Highest single impurity 0.08%
Melting point: 184-186°C

It was found not necessary to carry out high vacuum distillation to purify the citalopram when using potassium t-butoxide in step (a). Toluene was selected rather than the hazardous ether used in step (b) according to USP 4 136 193, and the base used in step (b) to adjust pH was ammonia, rather than NaOH.

### Comparative Example

Following the procedure of Example 1, but replacing the 72g potassium t-butoxide/575ml DMSO with 21g sodium hydride/900 ml DMSO in step (a), citalopram HBr was isolated:
Yield: 65g
Priority by HPLC: 99%
Highest single impurity: 0.16%

### Example 2: Isolation of Hiqh Purity Crystalline R,S-Citalopram using Potasium tertiary Butoxide as a Base

(a) The method of Example 1, step (a) is followed.
(b) Then, the acetic acid solution is diluted with 160ml of isopropyl alcohol, cooled to 5-10°C and the pH is then adjusted to 8.5-9.0 using aqueous ammonia (85ml) whilst maintaining the temperature between 5-10°C for 4 hours. The crystalline citalopram base thus produced is then filtered and washed with cold aqueous *iso*propyl alcohol (20%, 100ml) followed by washing with 100ml hexane. The product is dried at 40°C under a vacuum of 500-600mm Hg until at constant weight.

Yield: 95-97g
Purity by HPLC: 99.5%
Highest single impurity: 0.08%
Melting point: 89-91°C

The details of experimental data with solvents other than *iso*propyl alcohol/water are given in Table - 1

**Table - 1**

| **SI.No** | **Solvent** | **Solvent ratio (v/v)** | **Yield** | **Purity by HPLC** | **Highest single impurity** | **Melting point (°C)** |
|---|---|---|---|---|---|---|
| 1 | Water, Ethanol | 1:1 | 96g | 99.25% | 0.15% | 88-90 |
| 2 | Water, Methanol | 1:1 | 95g | 99.22% | 0.18% | 88-90 |
| 3 | Water, Dimethylformamide | 1:1 | 94g | 99.12%, | 0.24% | 88-90 |
| 4 | Water, Dimethylsulphoxide | 1:1 | 94g | 98.78%, | 0.26% | 87-89 |
| 5 | Water, Acetone | 1:1 | 94g | 98.94% | 0.16% | 87-89 |

The salient features of the process of Example 2 are elimination of sodium hydride and n-butyl lithium in the reaction, and isolation of pure citalopram hydrobromide with improved yield.

### Example 3: Isolation of High Purity Crystalline R,S-Citalopram using Sodium Hydride as a Base.

The procedure of Example 2 is followed, except in step (a), the potassium t-butoxide in DMSO is replaced by 21 grams of sodium hydride (60% in mineral oil) dissolved in 900ml of dimethyl sulphoxide.
Yield: 92-94g
HPLC purity: 99.32%
Highest single impurity: 0.12%
Melting point: 89-91°C

The above data show improved yield and purity over the corresponding process using prior art step (b).

The details of experimental data with solvents other than isopropyl alcohol/water are given in the Table - 2

**Table - 2**

| **SI. No** | **Solvents** | **Solvent ratio (v/v)** | **Yield** | **Purity by HPLC** | **Highest single impurity** | **Melting point (°C)** |
|---|---|---|---|---|---|---|
| 1 | Water, Ethanol | 1:1 | 91g | 99.25% | 0.15% | 88-90 |
| 2 | Water, Methanol | 1:1 | 91g | 99.22% | 0.17% | 88-90 |
| 3 | Water, Dimethylformamide | 1:1 | 92g | 98.32%, | 0.28% | 88-90 |
| 4 | Water, Dimethylsulphoxide | 1:1 | 92g | 98.78%, | 0.26% | 87-89 |
| 5 | Water, Acetone | 1:1 | 90g | 98.84% | 0.18% | 87-89 |

### Example 4: Preparation of Citalopram Hydrobromide from Crystalline Citalopram Base

100gms of crystalline citalopram base are dissolved in *iso*propyl alcohol (IPA, 600ml) at 50°-60°C. The clear solution is treated with activated carbon and then filtered. To the clear filtrate, aqueous hydrobromic acid (47%) is added below 5-10°C and the pH is adjusted to 1-2. The temperature (5-10°C) is maintained for an additional 6 hours. The precipitated solid is filtered, washed with 100mL IPA (0-5°C) followed by n-hexane (100mL) to produce pure crystalline citalopram hydrobromide.
Yield: 110-115gms
HPLC purity: > 99.8%

## Claims

1. A process for the preparation of citalopram, which process comprises the C-alkylation of 1-(4'-fluorophenyl)-1,3-dihydro*iso*benzofuran-5-carbonitrile (5-cyanophthalane) with 3-dimethylaminopropylchloride in the presence of a base, ***characterized in that*** the base is potassium tertiary butoxide.

2. A process according to claim 1, wherein the alkylation is carried out in the presence of a solvent.

3. A process according to claim 1 or claim 2, wherein the alkylation is carried out in the presence of dimethylsulphoxide (DMSO).

4. A process according to any preceding claim, wherein the alkylation is carried out at a temperature of from 25 to 40 °C.

5. A process according to any preceding claim, wherein an aqueous acid is added to the reaction mixture.

6. A process according to claim 5, wherein the aqueous acid is an aqueous organic acid, including acetic acid.

7. A process according to claim 5 or claim 6, wherein the citalopram thereby produced is isolated as a crystalline solid in one step from the reaction mixture.

8. A process for the preparation of crystalline citalopram, which process comprises crystallization thereof in one step from an aqueous mixture of oily citalopram.

9. A process according to any of claims 5 to 8, wherein an equal volume of a water-miscible solvent is added to the mixture.

10. A process according to any of claims 5 to 9, wherein an equal volume of a water-miscible solvent selected from: alcohols, such as methanol, ethanol and *iso*propanol (IPA); ketones, such as acetone; dimethylformamide (DMF) and/or dimethylsulphoxide (DMSO), is added to the mixture.

11. A process according to claim 9 or claim 10, wherein the water-miscible solvent is *iso*propylalcohol (IPA)).

12. A process for preparing citalopram hydrobromide, which process comprises reacting citalopram (base) with aqueous hydrobromic acid.

13. A process according to claim 12, wherein the citalopram (base) is prepared by a process according to any of claims 1 to 11.

14. A process according to claim 12 or claim 13, wherein the citalopram (base) is in a water-miscible solvent.

15. A process according to any of claims 12 to 14, wherein the pH of the reaction mixture is adjusted to acidic.

16. A process according to any of claims 12 to 15, wherein the pH of the reaction mixture is in the range of from 1 to 3, such as pH 1-2.

17. A process according to any of claims 12 to 16, wherein the aqueous hydrobromic acid is added at a temperature in the range of from 5-10°C.

## Amended claims

### Amended claims in accordance with Rule 86(2) EPC.

1. A process for the isolation of crystalline citalopram base from the C-alkylation reaction mixture of 1-(4'-fluorophenyl)-1,3-dihydro*iso*benzofuran-5-carbonitrile (5-cyanophthalane) and 3-dimethylaminopropylchloride in the presence of a base, comprising isolating the citalopram base as a crystalline solid directly from the reaction mixture.

2. A process according to claim 1, wherein the alkylation reaction is carried out in the presence of a solvent.

3. A process according to any of claims 2, wherein the alkylation reaction is carried out in the presence of dimethylsulphoxide (DMSO).

4. A process according to claim 3, wherein the alkylation is carried out at a temperature of from 25 to 40 °C.

5. A process according to any preceding claim, wherein said alkylation reaction mixture is diluted with water and extracted with a non-polar, organic solvent and back extracted with aqueous acid.

6. A process according to claim 5, wherein the aqueous acid is an aqueous organic acid, including acetic acid.

7. A process according to claim 5 or claim 6, wherein an equal volume of a water-miscible solvent is added to the aqueous organic acid extract.

8. A process according to claim 7, wherein an equal volume of a water-miscible solvent selected from: alcohols, such as methanol, ethanol and *iso*propanol (IPA); ketones, such as acetone; dimethylformamide (DMF) and/or dimethylsulphoxide (DMSO), is added to the mixture.

9. A process according to claim 8, wherein the water-miscible solvent is isopropylalcohol (IPA).

10. A process according to any of claims 7 to 9, wherein the pH is adjusted to about 8.5-9 using a base selected from aqueous ammonia, sodium hydroxide or carbonate or potassium hydroxide or carbonate, to precipitate crystalline citalopram base.

11. A process according to claim 10, wherein the pH is adjusted at between 10-15°C.

12. A process according to claim 10 or claim 11, wherein the crystalline citalopram base is filtered with aqueous water-miscible solvent.

13. A process according to claim 12, wherein the water-miscible solvent is isopropylalcohol (IPA).

14. A process according to claim 12 or claim 13, wherein the filtered crystalline citalopram base is washed with a washing agent to remove the water-miscible solvent.

15. A process according to claim 14, wherein the washing agent is hexane.

16. A process according to any preceding claim, further comprising reacting the crystalline citalopram with aqueous hydrobromic acid to prepare citalopram hydrobromide.
